# EUROPEAN PATENT SPECIFICATION

(11) **EP 2 752 494 B1**
(45) Date of publication and mention of the grant of the patent: **07.10.2020**
(21) Application number: 13199674.6
(22) Date of filing: 27.12.2013
(51) Int. Cl.: C12P 7/06, C12P 7/16, C12P 7/42, C12P 7/54

(54) **Method for enhancing carbon biofixation using lactic acid**
Verfahren zur Verbesserung von Kohlenbiofixierung mit Milchsäure
Procédé pour améliorer la biofixation de carbone à l'aide d'acide lactique

(30) Priority: 04.01.2013 US 201361749010 P; 26.12.2013 US 201314140627
(43) Date of publication of application: 09.07.2014
(73) Proprietor: Industrial Technology Research Institute, Hsinchu County 31040 (TW)
(72) Inventor: Chen, Chang-Chieh, Taipei City 111 (TW); Kuo, Hsin-Tzu, Kaohsiung City 800 (TW); Chou, Yi-Te, Taipei City 103 (TW); Hsu, Chin-Chen, Taipei City 106 (TW); Wu, Shao-Wen, Nantou County 545 (TW)
(74) Representative: Fuchs Patentanwälte Partnerschaft mbB

(56) References cited:
- EP-A1- 2 631 298
- US-A1- 2011 296 747
- M. KOPKE ET AL: "2,3-Butanediol Production by Acetogenic Bacteria, an Alternative Route to Chemical Synthesis, Using Industrial Waste Gas", APPLIED AND ENVIRONMENTAL MICROBIOLOGY, vol. 77, no. 15, 17 June 2011 (2011-06-17) , pages 5467-5475, XP055104754, ISSN: 0099-2240, DOI: 10.1128/AEM.00355-11
- MICHAEL KÖPKE ET AL: "Fermentative production of ethanol from carbon monoxide", CURRENT OPINION IN BIOTECHNOLOGY, vol. 22, no. 3, 1 June 2011 (2011-06-01), pages 320-325, XP55104855, ISSN: 0958-1669, DOI: 10.1016/j.copbio.2011.01.005
- WILLIAM E BALCH ET AL: "Acetobacterium, a New Genus of Hydrogen-Oxidizing, Carbon Dioxide-Reducing, Anaerobic Bacteria", INTERNATIONAL JOURNAL OF SYSTEMATIC BACTERIOLOGY, SOCIETY FOR GENERAL MICROBIOLOGY, READING, GB, vol. 27, no. 4, 1 October 1977 (1977-10-01), pages 355-361, XP002659783, ISSN: 0020-7713
- Jose Perez: "Reduction Of Carboxylic Acids To Alcohols Using Syngas And Clostridium Ljungdahlii As Biocatalyst", Theses and Dissertations, Cornell University, 20 August 2012 (2012-08-20), XP055110423, Retrieved from the Internet: URL:http://ecommons.library.cornell.edu/ha ndle/1813/31063 [retrieved on 2014-03-27]
- C. LEANG ET AL: "A Genetic System for Clostridium ljungdahlii: a Chassis for Autotrophic Production of Biocommodities and a Model Homoacetogen", APPLIED AND ENVIRONMENTAL MICROBIOLOGY, vol. 79, no. 4, 30 November 2012 (2012-11-30), pages 1102-1109, XP055110483, ISSN: 0099-2240, DOI: 10.1128/AEM.02891-12
- YAN NI ET AL: "A biocatalytic hydrogenation of carboxylic acids", CHEMICAL COMMUNICATIONS, vol. 48, no. 99, 1 January 2012 (2012-01-01), page 12056, XP055110404, ISSN: 1359-7345, DOI: 10.1039/c2cc36479d
- CHEN C-K ET AL: "Acetate enhances solvent production and prevents degeneration in Clostridium beijerinckii BA101", APPLIED MICROBIOLOGY AND BIOTECHNOLOGY, SPRINGER VERLAG, BERLIN, DE, vol. 52, 1 August 1999 (1999-08-01), pages 170-173, XP002678047, ISSN: 0175-7598
- PEREZ J M ET AL: "Biocatalytic reduction of short-chain carboxylic acids into their corresponding alcohols with syngas fermentation", BIOTECHNOLOGY AND BIOENGINEERING, WILEY & SONS, HOBOKEN, NJ, US, vol. 110, no. 4, 1 April 2013 (2013-04-01) , pages 1066-1077, XP002694318, ISSN: 0006-3592
- KAN LIU ET AL: "Mixed culture syngas fermentation and conversion of carboxylic acids into alcohols", BIORESOURCE TECHNOLOGY, vol. 152, 1 January 2014 (2014-01-01), pages 337-346, XP055110212, ISSN: 0960-8524, DOI: 10.1016/j.biortech.2013.11.015
- BANERJEE A. ET AL.: "LACTOSE-INDUCIBLE SYSTEM FOR METABOLIC ENGINEERING OF CLOSTRIDIUM LJUNGDAHLII", APPLIED AND ENVIRONMENTAL MICROBIOLOGY, vol. 80, no. 8, April 2014 (2014-04), pages 2410-2416, XP008168501,
- BRAUN K ET AL: "EFFECT OF MOLECULAR HYDROGEN AND CARBON DI OXIDE ON CHEMO ORGANOTROPHIC GROWTH OF ACETOBACTERIUM-WOODII AND CLOSTRIDIUM-ACETICUM", ARCHIVES OF MICROBIOLOGY, SPRINGER BERLIN HEIDELBERGRG, vol. 128, no. 3, 1 January 1981 (1981-01-01), pages 294-298, XP008181676, ISSN: 0302-8933, DOI: 10.1007/BF00422533

## Description

Recent climate anomalies, including hurricanes and droughts, have been associated with global warming, i.e., an increase in global temperature. A major cause of the global warming has been attributed to the increase in atmospheric CO₂ concentration. Indeed, it has been reported that the atmospheric CO₂ concentration increased from around 280 ppm to 368 ppm over the past 200 years. Thus, there is an ongoing interest in developing processes that could efficiently reduce CO₂ emission or capture CO₂ from the atmosphere.

Processes using living organisms to convert CO₂ into organic compounds are generally referred to as carbon dioxide biofixation. The most prominent example of carbon dioxide biofixation involves the use of photosynthetic microorganisms, such as microalgae and cyanobacteria, which use energy from sunlight to drive a carbon fixation pathway.

Besides photosynthetic microorganisms, chemoautotrophic microorganisms (ones that obtain their nourishment through the oxidation of inorganic chemical compounds as opposed to photosynthesis) represent a possible alternative for use in carbon biofixation processes.

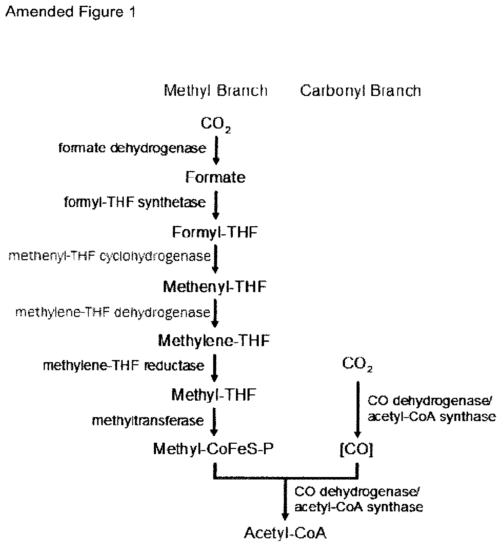

A major non-photosynthetic carbon dioxide fixation pathway used by chemoautotrophic microorganisms is the Wood-Ljungdahl (WL) pathway (Figure 1). The WL pathway is commonly employed by anaerobic organisms, which include *Clostridium aceticum, Clostridium autoethanogenum, Clostridium carboxidivorans, Clostridium difficile, Clostridium ljungdahlii, Moorella thermoacetica* (formerly *Clostridium thermoaceticum*), *Methanobacterium thermoautotrophicum, Defulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii.*

Although some acetogenic *Clostridia* have been engineered to produce commodity chemicals (e.g., butyrate, acetate, acetoin, and acetone) and biofuels (e.g., butanol and ethanol), there remains a need to develop methods that may improve anaerobic organisms' ability to metabolize CO₂ into useful chemicals and/or biofuels.

Accordingly, provided herein is the use of lactic acid and/or salts thereof for inducing 1.5 fold or more increase, relative to the microorganism cultured without the presence of lactic acid and/or salts thereof, of at least one gene chosen from codH, codH beta, codH delta, codH gamma, coos 1, and metF in a process for microbial fermentation of a gaseous substrate comprising CO₂, the processes comprising:
providing a gaseous substrate and a liquid medium to a microorganism chosen from *Clostridium aceticum, Clostridium autoethanogenum, Clostridium carboxidivorans, Clostridium difficile, Clostridium ljungdahlii, Moorella thermoacetica* (formerly *Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii,* wherein the liquid medium comprises lactic acid and/or salts thereof at a concentration ranging from 0.5 g/L to 3 g/L,, and wherein the gaseous substrate comprises at least 10% by volume of CO₂, and
allowing the microorganism to produce at least one product chosen from acids and alcohols.

Also provided herein is the use of lactic acids and/or salts thereof for inducing 1.5 fold or more increase relative to a microorganism cultured without the presence of lactic acid and/or salts thereof, of at least one gene chosen from codH, codH beta, codH delta, codH gamma, coos 1 and metF in a process of improving efficiency of carbon capture in microbial fermentation, comprising:
providing a gaseous substrate and a liquid medium to a microorganism chosen from *Clostridium aceticum*, *Clostridium autoethanogenum, Clostridium carboxidivorans, Clostridium difficile, Clostridium ljungdahlii, Moorella thermoacetica* (formerly *Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii,* wherein the liquid medium comprises lactic acid and/or salts thereof at a concentration ranging from 0.5 g/L to 3 g/L, and wherein the gaseous substrate comprises at least one carbon source chosen from CO and CO₂, wherein the gaseous substrate comprises at least 10% by volume of CO₂, and
allowing the microorganism to produce at least one product chosen from acids and alcohols.

The accompanying drawings, which are incorporated in and constitute a part of this specification, illustrate exemplary embodiments of the present disclosure.

### BRIEF DESCRIPTION OF THE DRAWINGS

Figure 1 shows the Wood-Ljungdahl (WL) pathway.
Figure 2 shows a growth curve of *Clostridium ljungdahlii* BCRC 17797, exposed to CO₂ and H₂ gas mixture, in (a) experimental group (containing 2.5 g/L of sodium lactate in the medium), (b) sodium ion control group (containing 1.52 g/L of NaCl in the medium), and (c) no-additive control group, as described in the Example section.
Figure 3 shows that lactate permease and certain genes involved in the Wood-Ljungdahl pathway may be induced by the presence of 2.5 g/L sodium lactate in the culture medium. The fold increases were in comparison with the no-additive control group, as described in the Example section.
Figure 4 shows that lactate permease and certain genes involved in the Wood-Ljungdahl pathway may be induced by the presence of 2.5 g/L sodium lactate in the culture medium. The fold Increases were in comparison with the sodium-ion control group, as described in the Example section.
Figure 5 shows the expressions of certain genes, relative to the no-additive control group, in the two experimental groups (1 g/L and 2.5 g/L of sodium lactate), as described in the Example section.
Figure 6 shows, after 96 hours of culture, the amount of acetic acid produced in the experimental groups, the no-additive control group (syngas only), and the sodium ion control group, as described in the Example section.
Figure 7 shows, after 96 hours of culture, the amount of ethanol produced in the experimental groups, the no-additive control group (syngas only), and the sodium ion control group, as described in the Example section.
Figure 8 shows, after 90 hours of culture, the expressions of certain genes, relative to the no-additive control group, in the experimental group containing 1 g/L of sodium lactate, as described in the Example section.

### DESCRIPTION OF THE EMBODIMENTS

Below, exemplary embodiments will be described in detail with reference to accompanying drawings so as to be understood by a person having ordinary skill in the art.

Provided herein is the use of lactic acids and/or salts thereof for inducing 1.5 fold or more increase, relative to a microorganism cultured without the presence of lactic acid and/or salts thereof, of at least one gene chosen from codH, codH beta, codH delta, codH gamma, coos 1, and metF in a process for microbial fermentation of a gaseous substrate comprising CO₂, the processes comprising:
providing a gaseous substrate and a liquid medium to a microorganism chosen from *Clostridium aceticum, Clostridium autoethanogenum, Clostridium carboxidivorans, Clostridium difficile, Clostridium ljungdahlii, Moorella thermoacetica* (formerly *Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii,* wherein the liquid medium comprises lactic acid and/or salts thereof at a concentration ranging from 0.5 g/L to 3 g/L, and wherein the gaseous substrate comprises at least 10% by volume of CO₂, and
allowing the microorganism to produce at least one product chosen from acids and alcohols.

Also provided herein is the use of lactic acid and/or salts thereof for inducing 1.5 fold or more increase, relative to a microorganism cultured without the presence of lactic acid and/or salts thereof, of at least one gene chosen from codH, codH beta, codH delta, codH gamma, coos 1 and metF in a process of improving efficiency of carbon capture in microbial fermentation, comprising:
providing a gaseous substrate and a liquid medium to a microorganism chosen from *Clostridium aceticum, Clostridium autoethanogenum, Clostridium carboxidivorans, Clostridium difficile, Clostridium ljungdahlii, Moorella thermoacetica* (formerly *Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii,* wherein the liquid medium comprises lactic acid and/or salts thereof at a concentration ranging from 0.5 g/L to 3 g/L, and wherein the gaseous substrate comprises at least one carbon source chosen from CO and CO₂, wherein the gaseous substrate comprises at least 10% by volume of CO₂, and
allowing the microorganism to produce at least one product chosen from acids and alcohols.

In some embodiments, the microorganism is chosen from photosynthetic microorganisms genetically engineered to be capable of converting CO₂ and/or CO into at least one product chosen from acids and alcohols via Wood-Ljungdahl (WL) pathway.

The microorganism is chosen from *Clostridium aceticum, Clostridium autoethanogenum, Clostridium carboxidivorans, Clostridium difficile, Clostridium ljungdahli, Moorella thermoacetica (formerly Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui* and *Acetobacterium woodii.*

The term "gaseous substrate comprises CO₂" and like terms should be understood to include any gaseous substrate in which carbon dioxide (CO₂) is available to the microorganism for growth and/or fermentation. Similarly, the term "the gaseous substrate comprises at least one carbon source chosen from CO and CO₂" should be understood to include any gaseous substrate in which CO₂ and/or CO is available to the microorganism for growth and/or fermentation.

The gaseous substrate for use in the invention comprises at least 10% by volume of CO₂. Such substrate may be a CO₂ and/or CO- containing waste gas obtained as a by-product of an industrial process, or from another source such as from automobile exhaust fumes. Exemplary industrial processes include but not limited to: petroleum refining processes, gasification of coal, electric power production, carbon black production, ammonia production, methanol production, and coke manufacturing. Depending on the composition of the CO₂, or CO₂ and CO-containing gaseous substrate, it may be desirable, prior to introducing it to the fermentation, to remove undesired impurities, such as dust particles from the gaseous substrate. For example, the gaseous substrate may be filtered or scrubbed using known methods.

In some embodiments, the gaseous substrate may further comprise at least one gas chosen from hydrogen (H₂) and nitrogen (N₂).

In some embodiments, the gaseous substrate is a synthesis gas ("syngas") comprising CO₂, CO, and H₂. Syngas can be produced by steam reforming of natural gas or gasification of various organic materials such as biomass, organic waste, coal, petroleum, plastics, or other carbon containing materials.

The gaseous substrate comprises at least 10% by volume of CO₂. In some embodiments it comprises at least 20%, at least 30%, at least 40%, at least 50%, at least 60%, at least 70%, at least 80%, or at least 90% by volume of CO₂. In some embodiments, the gaseous substrate may comprise from 20% to 80% of CO₂. As a non-limiting example, the gaseous substrate may comprise 20% of CO₂ and 80% of H₂.

In general, CO₂, or CO₂ and CO will be provided to the microorganism in a gaseous state. However, in some embodiments, CO₂, or CO₂ and CO can be provided in a liquid. For example, a liquid may be saturated with the gaseous substrate comprising CO₂ and that liquid is then added to a bioreactor.

In some embodiments, reduced chemical species and/or electrode(s) may be provided to the microorganism as an electron source for CO₂ fixation.

In some embodiments, at least one electron source may be provided to the microorganism for CO₂ fixation, wherein the at least one electron source may be chosen from hydrogen (H₂), reduced carbon compounds (as non-limiting examples: CO, formate, and methanol), sulfur compounds (as non-limiting examples: H₂S and S), and nitrogen compounds (as a anon-limiting example, NH₃).

The liquid medium provided to the microorganism comprises 0.5 g/L to 3 g/L lactic acid and/or salts thereof. As non-limiting examples, the liquid medium provided to the microorganism may comprise, for instance, 0.5 g/L, 1 g/L, 1.25 g/L, 1.5 g/L, 1.75 g/L, 2 g/L, 2.25 g/L, 2.5 g/L, 2.75 g/L, or 3 g/L lactic acid and/or salts thereof.

The lactic acid and/or salts thereof is present in an amount sufficient to induce the expression of at least one gene chosen from CLJU_c37670, CLJU_c37570, CLJU_c37550. CLJU_c37580, CLJU_c06990, CLJU_c20040, CLJU_c08930, CLJU_c07020, CLJU_c37650, CLJU_c37640, CLJU_c37630, CLJU_c37610, CLJU_c37560, and CLJU_c09110, involved in the Wood-Ljungdahl pathway as described in Köpke M. et al., Clostridium ljungdahlii represents a microbial production platform based on syngas. (2010).

The lactic acid and/or salts thereof is present in an amount sufficient to induce about a 1.5 fold or more increase, relative to the microorganism cultured under the same condition but without the presence of lactic acid/or salts thereof, of at least one gene involved in the Wood-Ljungdahl pathway, namely at least one gene chosen from codH, codH beta, codH delta, codH gamma, coos1, and metF.

In some embodiments, the lactic acid and/or salts thereof may be present in the liquid medium in an amount sufficient to induce about a 1.5 fold to about a 10 fold increase, relative to the microorganism cultured under the same condition but without the presence of lactic acid and/or salts thereof, of at least one gene chosen from codH, codH beta, codH delta, codH gamma, coos1 and metF. As a non-limiting example, the lactic acid and/or salts thereof may be present in the liquid medium in an amount sufficient to induce about a 1.5 fold to about an 8 fold increase, relative to the microorganism cultured under the same condition but without the presence of lactic acid and/or salts thereof, of at least one gene chosen from codH, codH beta, codH delta, codH gamma, coos1 and metF.

To increase the growth of the microorganism and/or fixation of CO₂, and optionally CO, in addition to lactic acid and/or salts thereof, the liquid medium may be supplemented with additional nutrients or ingredients suitable for growing the bacteria. For example, the medium used for culturing the microorganism may also comprise vitamins, salts, extracts, and/or minerals sufficient to permit growth of the microorganism.

The process for CO₂ and optionally CO fixation is carried out under conditions for the desired fermentation to occur (e.g., CO₂ to acids). Reaction conditions that can be considered include pressure, temperature, gas flow rate, liquid flow rate, media pH, media redox potential, agitation rate (if using a continuous stirred tank reactor), inoculum level, maximum gas substrate concentrations to ensure that CO₂ or other components in the liquid phase does not become limiting, and maximum product concentrations to avoid product inhibition.

In some embodiments, the processes may be carried out in any suitable bioreactor in which the substrate can be contacted with one or more microorganisms, such as a continuous stirred tank reactor, an immobilized cell reactor, a gas-lift reactor, a bubble column reactor, a membrane reactor such as a Hollow Fiber Membrane Bioreactor or a trickle bed reactor, monolith bioreactor, or loop reactors.

In some embodiments, fermentation processes described herein may produce products including but not limited to alcohols and/or acids. Exemplary acids include but are not limited to formic acid, acetic acid, propionic acid, butyric acid, acrylic acid, fatty acids; and exemplary alcohols include but are not limited to methanol, ethanol, propanol, butanol, and 2,3 butanediol. In some embodiments, fermentation processes described herein may produce products including but not limited to acetone.

In the context of fermentation products, the term "acid" as used herein includes both carboxylic acids and the associated carboxylate anion. For example, the term "acetic acid" includes free acetic acid, acetate salt alone, and a mixture of free acetic acid and acetate salt.

The term "fatty acid" includes any carboxylic acids with a long aliphatic chain. In some embodiments, the aliphatic chain can be linear or branched, and saturated or unsaturated. In some embodiments, the aliphatic chain may contain, for example, from 4 to 12 carbon atoms, 5 to 11 carbon atoms, or 8 to 10 carbon atoms.

At least one product chosen from acids and alcohols of the fermentation processes may be recovered using any known methods.

As a non-limiting example, ethanol may be recovered from a fermentation broth by methods such as fractional distillation or evaporation, and extractive fermentation.

Also as a non-limiting example, acetate may be recovered from a fermentation broth by using an adsorption system involving an activated charcoal filter. In this case, a suitable separation unit may first be used to remove microorganisms from the fermentation broth. The cell free acetate-containing medium is then passed through a column containing activated charcoal to adsorb the acetate. Acetate in the acid form (acetic acid) rather than the salt (acetate) form is more readily adsorbed by activated charcoal. Thus, the pH of the cell free acetate-containing medium may be reduced to less than about 3 before it is passed through the activated charcoal column, to convert the majority of the acetate to the acetic acid form.

Further as a non-limiting example, butyric acid present in a fermentation broth may be recovered and purified by extraction using an aliphatic amine, such as Alamine 336 (tri-octyl/decyl amine; Alamine336®, Cognis, Cincinnati, OH, USA), or other water-immiscible solvents.

### EXAMPLE

### Example 1

### Microorganism

*Clostridium ljungdahlii* BCRC 17797 (DSM No. 13528; BCRC No. 17797) was purchased from Bioresource Collection and Research Center, Taiwan. This strain was known to grow and generate metabolites by utilizing the carbon source in the atmosphere, such as CO, gas mixture of CO₂ and H₂, or synthesis gas (CO and H₂), through the Wood-Ljungdahl pathway (Figure 1). See Köpke M. et al., Clostridium ljungdahlii represents a microbial production platform based on syngas, Proc Natl Acad Sci USA, 107(29), pp. 13087-13092 (2010).

### Materials

Modified PETC Basal Medium containing (per liter): protease peptone (10 g), yeast extract (3 g), and resazurin stock solution (0.5 mL, 0.1%, w/v). See Cottera JL et al, Influence of process parameters on growth of Clostridium ljungdahlii and Clostridium autoethanogenum on synthesis gas, Enzyme and Microbial Technology 44(5), pp. 281-299 (2008).

ATCC 1754 Salt Solution containing (per liter): NH₄Cl (20 g), KCI (2 g), MgSO₄·7H₂O (4 g), NaCl (16 g), KH₂PO₄ (2 g), and CaCl₂ (0.4 g).

Vitamin Solution containing (per liter): biotin (2 mg), folic acid (2 mg), pyridoxine (10 mg), thiamine HCl (5 mg), riboflavin (5 mg), nicotinic acid (5 mg), calcium D(+) pantothenate (5 mg), vitamin B12 (5 mg), p-aminobenzoic acid (5 mg), and lipoic acid (thioctic) (5 mg).

Trace Element Solution containing (per liter): nitrilotriacetic acid (2 g), MnCl₂·4H₂O (1.3 g), FeSO₄·7H₂O (0.4 g), CoCl₂·6H₂O (0.2 g), ZnSO₄·7H₂O (0.2 g), CuCl₂·2H₂O (0.02 g), NiCl₂·6H₂O (0.02 g), Na₂MoO₄·2H₂O (0.02 g), Na₂SeO₃ (0.02 g), and Na₂WO₄·2H₂O (0.025 g).

Modified PETC Medium (mPETC), pH 6.8: To prepare mPETC, one liter of Modified PETC Basal Medium was supplemented with 50 ml of ATCC 1754 Salt Solution, 10 ml of Vitamin Solution, and 10 ml of Trace Element Solution. The prepared medium was then dispensed into serum bottles and then autoclaved at 121 °C, 15 psi (∼0.103 MPa), for 15-20 minutes.

Air-tight bottles used for anaerobic culturing: 200 ml crimp-top serum bottle sealed with a rubber stopper, which was further held in place by an aluminum crimp to ensure an air-tight closure.

### Methods

*Clostridium ljungdahlii* was first cultured anaerobically with mPETC medium containing 10 g/L of fructose at 37 °C in one of the air-tight bottles mentioned above. When OD₆₀₀ reached a value ranging from 1 to 1.5, the culture medium was centrifuged at 3500 rpm for 210 seconds. The resulting cell pellet was resuspended with mPETC without fructose and then further cultured anaerobically at 37 °C for one hour to remove any residual sugar substrates.

45 ml of mPETC culture medium was added to one of the crimp-top serum bottles mentioned above, which was then covered with an aluminum foil and autoclaved for 15 minutes at 121 °C, 15 psi (∼0.103 MPa). After cooling, the medium-containing bottle was moved into an anaerobic chamber, and 50 µl of cysteine-HCI (50% w/v) was added to the medium to remove any dissolved oxygen.

For an experimental group, sodium lactate solution was further added to the culture medium to obtain a lactate concentration of 2.5 g/L (about 0.022 M).

For a sodium ion control group, NaCl solution was added to the culture medium to obtain a NaCl concentration of 1.52 g/L (about 0.026 M). This control group was designed to test whether the sodium ions present in the sodium lactate play any role in carbon dioxide fixation. Thus, the same molar amount of sodium ion as the experimental group was added.

For a no-additive control group, an appropriate amount of water was added to the culture medium.

Each of the serum bottles containing 45 ml of mPETC culture medium was then inoculated with 5 ml of *Clostridium ljungdahlii* culture that had residual sugar removed. A sample taken at this point was labeled as t=0 sample. The bottles were then sealed with rubber stoppers and further covered with aluminum crimp caps to ensure an air-tight closure.

The headspaces of the air-tight bottles were first purged with a gas mixture containing 80% N₂ and 20% of CO₂ under a pressure of 10 psi (∼ 0.068 MPa). Needles were inserted into the rubber stops to allow the gas to escape from the bottles. After about 30 seconds of exposure to the gas mixture, an anaerobic condition filled with CO₂ was obtained within the bottles.

Using needles, the headspaces of the bottles were then further purged with another gas mixture containing 80% H₂ and 20% of CO₂ under a pressure of 20 psi (∼0.137 MPa) to replace the previous gas mixture. After about 30 seconds, needles were removed and the pressure within the bottles was built up to about 20 psi (∼0.137 MPa). The bottles were then moved to a 37 °C incubator and placed on a shaker providing a shaking speed of 100 rpm.

Every 12 hours, a needle was used to release the gas built up within the bottle, and the headspace of the bottle was purged and refreshed under the same process as described above to replenish gaseous carbon source under a stabilized pressure.

Syringes with needles were used to collect liquid samples. After measuring the OD₆₀₀, the liquid samples were centrifuged at 12,000 rpm for 10 minutes. The resulting cell pellet was stored in 700 µl TRIzol solution for later RNA extraction and gene expression analysis. The supernatant was analyzed with an Agilent 1100 series high-performance liquid chromatography (HPLC) with Aminex HPX-87 H (300 mm x 7.8 mm) column.

### Results

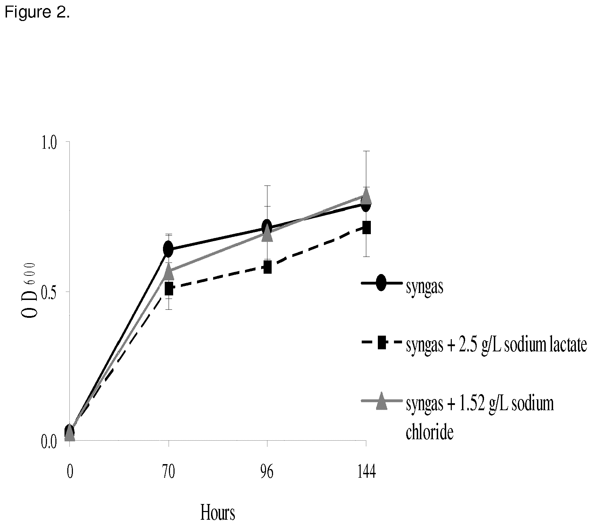

Figure 2 shows the changes of OD₆₀₀, an indication of bacterial growth, in the culture medium of (a) the experimental group (containing 2.5 g/L of sodium lactate), (b) the sodium ion control group (containing 1.52 g/L of NaCl), and (c) the no-additive control group. For all of the groups, *Clostridium ljungdahlii* BCRC 17797 grew rapidly during the first 70 hours, and their growth rates then slowed down.

At t=144 hours, the OD₆₀₀ value of: (a) the experimental group with sodium lactate reached 0.71, (b) the sodium ion control group reached 0.82, and (c) the non-additive control group reached 0.79. Accordingly, no significant difference was observed in the growth rates of these three groups.

Medium samples taken at t=144 hours were analyzed by HPLC. As shown in Table 1, when CO₂ was supplied as the carbon source, *Clostridium ljungdahlii* BCRC 17797 grown in mPETC was capable of producing acetic acid.

As shown in Table 1, at t=144 hours, the concentration of acetic acid in the experimental group was higher than that in either the no-additive control group or the sodium ion control group. And the concentration of acetic acid in the no-additive control group at t=144 hours was about the same as that of the sodium-ion control group.

**Table 1**

| Component | Group | C_{t=0 h} | C_{t=144 h} | ΔC (C_{t=144 h}-Cₜ₌₀) |
|---|---|---|---|---|
| Acetic Acid | No-additive control group (Syngas + mPETC) | 0.05 ± 0.00 | 5.02 ± 1.82 | 4.97 |
| | Sodium-ion control group (Syngas + 1.52 g/L sodium chloride in mPETC) | 0.06 ± 0.00 | 4.97 ± 0.82 | 4.91 |
| | Experimental group (Syngas + 2.5 g/L sodium lactate in mPETC) | 0.06 ± 0.00 | 6.45 ± 0.43 | 6.39 |
| Lactic acid | Experimental group (Syngas + 2.5 g/L sodium lactate in mPETC) | 2.92 ± 0.26 | 2.25 ± 0.17 | -0.67 |

As the data in Table 1 further shows, the concentration of lactic acid in the experimental group decreased from 2.92 g/L (at t=0) to 2.25 g/L (at t=144). This indicates that a small amount of lactic acid (i.e., 0.67 g/L) was consumed by the bacteria. 0.67 g/L of lactic acid consumed could provide about 0.022 M of carbon atoms, and 6.39 g/L of acetic acid produced in the experimental group contained about 0.213 M of carbon atoms. This indicates that at least 0.191 M (0.213 M minus 0.022 M) of carbon atoms came from CO₂ presented in the gas mixture.

As Table 1 above shows, after 144 hours of culture, the non-additive control group produced about 4.97 g/L of acetic acid, which contained about 0.166 M of carbon atoms. The sodium-ion control group produced about 4.91 g/L of acetic acid, which contained about 0.164 M of carbon atoms. Thus, the amount of CO₂ converted (fixed) to acetic acid by the experiment group (0.191 M (carbon atoms presented in the acetic acid produced by the experimental group minus the amount of carbon possibly converted from lactic acid) was at least about 15% higher than that of the non-additive control group (0.166 M of carbon atoms presented in the acetic acid produced in the non-additive control group) or the sodium-ion control group (0.164 M of carbon atoms presented in the acetic acid produced in the sodium-ion control group). This shows that lactic acid present in the culture medium of the experimental group improved the carbon dioxide fixation of *Clostridium ljungdahlii* BCRC 17797.

Real-time quantitative polymerase chain reaction (RT-qPCR) was used to quantify the expression of certain genes listed in Table 2.

**Table 2**

| Gene | Protein Expressed | code |
|---|---|---|
| lactate permease | lactate permease (the enzyme for transporting lactic acid into the cell) | CLJU_c21610 |
| codH | codH/CO dehydrogenase | CLJU_c37670 |
| codHbeta | CO dehydrogenase / acetyl-CoA synthase beta subunit | CLJU_c37550 |
| codHdelta | CO dehydrogenase / acetyl-CoA synthase delta subunit | CLJU_c37580 |
| codHgamma | CO dehydrogenase / acetyl-CoA synthase gamma subunit | CLJU_c37570 |
| cooS1 | anaerobic-type carbon monoxide dehydrogenase | CLJU_c09110 |
| metF | methylenetetrahydrofolate reductase | CLJU_c37610 |

| | | |
|---|---|---|
| Note: CO dehydrogenase is one of the enzymes that the bacteria utilized in converting CO₂ gas via the WL pathway; it is also an enzyme for the synthesis of acetyl-CoA. | | |

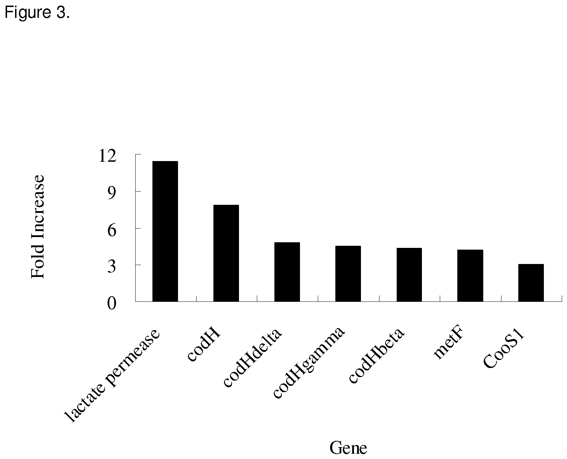

As shown in Figure 3, the presence of lactic acid in the culture medium of the experimental group induced, relative to the no-additive control group: (i) about an 11 fold increase of lactate permease, indicating that the bacteria was responsive to the lactic acid, (ii) about an 4 to 8 fold increase of codH, codH beta, codH delta, and codH gamma, (iii) about a 3 fold increase of cooS1; and (iv) about a 4 fold increase of metF.

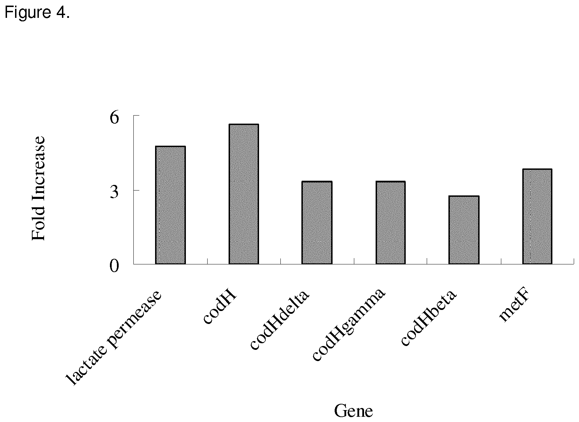

As shown in Figure 4, the presence of lactic acid in the culture medium of the experimental group induced, relative to the sodium-ion control group, about a 3 to 6 fold increase of lactate permease, codH, codH beta, codH delta, codH gamma, and metF. This indicates that lactic acid indeed may induce the expression of genes involved in carbon dioxide fixation.

### Example 2

The experimental protocol as described in Example 1 was repeated, except that, besides having a no-additive control group, two experimental groups were prepared, one had 1 g/L of lactate in the culture medium and the other had 2.5 g/L of lactate in the culture medium.

Table 3 below shows, after 96 hours of culture, the amount of lactate consumed and acetate produced for the experimental groups and a no-additive control group.

**Table 3**

| | | | |
|---|---|---|---|
| Lactate addition in medium (g/L) | 0 | 1 | 2.5 |
| **Lactate consumption (g/L)** | **0.00±0.00** | **0.23±0.03** | **0.23±0.01** |
| **Acetate production (g/L)** | **7.23±1.42** | **8.13±1.59** | **9.15±0.06** |

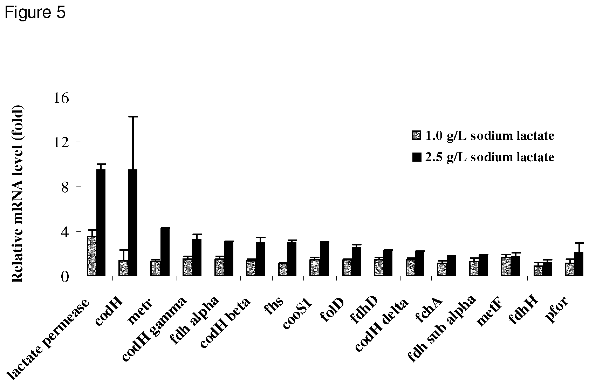

Figure 5 shows the expressions of certain genes, relative to the no-additive control group, in the two experimental groups.

### Example 3

The experimental protocol as described in Example 1 was repeated, except that, besides having a no-additive control group, three experimental groups (0.5 g/L, 1 g/L, and 3 g/L sodium lactate) and one sodium ion control group (containing 1.57 g/L of NaCl) were prepared.

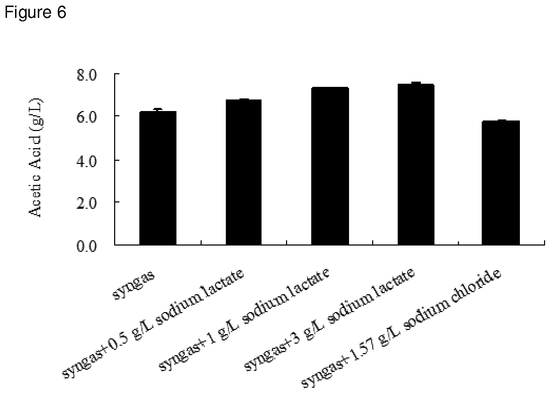

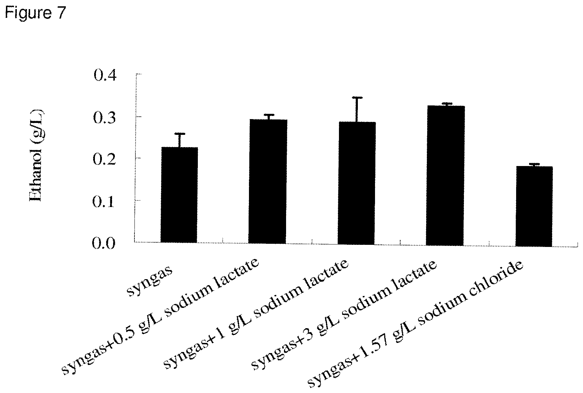

Figure 6 and 7 show, after 96 hours of culture, the amount of acetic acid and ethanol, respectively, produced in the experimental groups, the no-additive control group (syngas only), and the sodium ion control group.

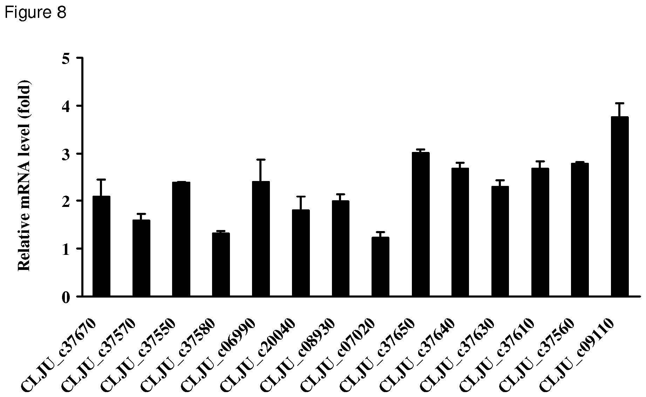

Figure 8 shows, after 90 hours of culture, the expressions of certain genes, relative to the no-additive control group, in the experimental group containing 1 g/L of sodium lactate. Table 4 below shows what enzyme each CLJU_c numbers shown in Figure 8 represents.

**Table 4**

| | |
|---|---|
| CLJU_ c37670 | **codH,** carbon monoxide dehydrogenase |
| CLJU_c37570 | **codH gamma**, CO dehydrogenase/acetyl-CoA synthase subunit gamma |
| CLJU_c37550 | **codH beta,** CO dehydrogenase/acetyl-CoA synthase complex subunit beta |
| CLJU_c37580 | **codH delta,** CO dehydrogenase/acetyl-CoA synthase subunit delta |
| CLJU_c06990 | **fdh alpha,** formate dehydrogenase subunit alpha |
| CLJU_ c20040 | **fdh sub alpha,** formate dehydrogenase subunit alpha |
| CLJU_c08930 | **fdhH,** formate dehydrogenase H |
| CLJU c07020 | **fdhD,** formate dehydrogenase subunit |
| CLJU_ c37650 | **fhs,** formate-tetrahydrofolate ligase |
| CLJU c37640 | **fchA,** formyltetrahydrofolate cyclohydrolase |
| CLJU c37630 | **folD,** bifunctional methylenetetrahydrofolate dehydrogenase/methenyltetrahydrofolate cyclohydrolase |
| CLJU_ c37610 | **metF,** methylenetetrahydrofolate reductase |
| CLJU_c37560 | **meTr,** methyltetrahydrofolate:corrinoid/iron-sulfur protein methyltransferase |
| CLJU c09110 | **cooS1,** anaerobic-type carbon monoxide dehydrogenase |

## Claims

1. Use of lactic acid and/or salts thereof for inducing 1.5 fold or more increase of the expression, relative to the microorganism cultured without the presence of lactic acid and/or salts thereof, of at least one gene chosen from codH (carbon monoxide dehydrogenase/acetyl-coenzyme A synthase), codH beta (carbon monoxide dehydrogenase/acetyl-coenzyme A synthase beta subunit), codH delta (carbon monoxide dehydrogenase/acetyl-coenzyme A synthase delta subunit), codH gamma (carbon monoxide dehydrogenase/acetyl-coenzyme A synthase gamma subunit), coos1 (anaerobic-type carbon monoxide dehydrogenase), and metF (methylenetetrahydrofolate reductase) in a process for microbial fermentation of a gaseous substrate comprising CO₂, the process comprising:
providing a gaseous substrate and a liquid medium to a microorganism chosen from *Clostridium aceticum, Clostridium autoethanogenum, Clostridium carboxidivorans, Clostridium difficile, Clostridium ljungdahli, Moorella thermoacetica (formerly Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii,* wherein the liquid medium comprises lactic acid and/or salts thereof at a concentration ranging from 0.5 g/L to 3 g/L, and wherein the gaseous substrate comprises at least 10% by volume of CO₂; and
allowing the microorganism to produce at least one product chosen from acids and alcohols.

2. The use according to claim 1, wherein the gaseous substrate further comprises at least one gas chosen from H₂, N₂, and CO.

3. The use according to claim 1 or 2, wherein the gaseous substrate comprises at least 20% by volume of CO₂.

4. The use according to at least one of the preceding claims, wherein the at least one product is chosen from acetic acid, propionic acid, butyric acid, acrylic acid, and fatty acid.

5. The use according to at least one of the preceding claims, wherein the at least one product is chosen from ethanol, acetone, propanol, butanol, and 2,3 butanediol.

6. The use according to at least one of the preceding claims, wherein the microorganism is capable of converting CO₂ and/or CO into at least one product chosen from acids and alcohols.

7. Use of lactic acid and/or salts thereof for inducing 1.5 fold or more increase of the expression, relative to the microorganism cultured without the presence of lactic acid and/or salts thereof, of at least one gene chosen from codH, codH beta, codH delta, codH gamma, coos1, and metF in a process of improving efficiency of carbon capture in microbial fermentation, comprising:
providing a gaseous substrate and a liquid medium to a microorganism chosen from *Clostridium aceticum, Clostridium autoethanogenum, Clostridium carboxidivorans, Clostridium difficile, Clostridium ljungdahli, Moorella thermoacetica (formerly Clostridium thermoaceticum), Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui,* and *Acetobacterium woodii,* wherein the liquid medium comprises lactic acid and/or salts thereof at a concentration ranging from 0.5 g/L to 3 g/L, and wherein the gaseous substrate comprises at least one carbon source chosen from CO₂ and optionally also CO, wherein the gaseous substrate comprises at least 10% by volume of CO₂; and
allowing the microorganism to produce at least one product chosen from acids and alcohols.

8. The use according to claim 7, wherein the gaseous substrate further comprises at least one gas chosen from H₂ and N₂.

9. The use according to claim 7 or 8, wherein the gaseous substrate comprises at least 20% by volume of CO₂.

10. The use according to at least one of the preceding claims, wherein the at least one product is chosen from acetic acid, propionic acid, butyric acid, acrylic acid, and fatty acid.

11. The use according to at least one of the preceding claims, wherein the at least one product is chosen from ethanol, acetone, propanol, butanol, and 2,3 butanediol.

12. The use according to at least one of the preceding claims, wherein the microorganism is capable of converting CO₂ and/or CO into at least one product chosen from acids and alcohols.

## Patentansprüche

1. Verwendung von Milchsäure und/oder Salzen davon zum Induzieren von 1,5-fachem Anstieg oder mehr der Expression, relativ zu dem Mikroorganismus, welcher ohne die Gegenwart von Milchsäure und/oder Salzen davon kultiviert wird, von mindestens einem Gen, ausgewählt aus codH (Kohlenmonoxid-Dehydrogenase/Acetyl-Coenzym A-Synthase), codH beta (Kohlenmonoxid-Dehydrogenase/Acetyl-Coenzym A-Synthase, beta-Untereinheit), codH delta (Kohlenmonoxid-Dehydrogenase/Acetyl-Coenzym A-Synthase, delta-Untereinheit), codH gamma (Kohlenmonoxid-Dehydrogenase/Acetyl-Coenzym A-Synthase, gamma-Untereinheit), coosl (Kohlenmonoxid-Dehydrogenase, anaerober Typ) und metF (Methylentetrahydrofolat-Reduktase), in einem Verfahren für mikrobielle Fermentation eines gasförmigen Substrats, umfassend CO₂, wobei das Verfahren umfasst:
Bereitstellen eines gasförmigen Substrats und eines flüssigen Mediums für einen Mikroorganismus, welcher aus *Clostridium aceticum, Clostridium autoethanogenum, Clostridium carboxidivorans, Clostridium difficile, Clostridium ljungdahli, Moorella thermoacetica* (früher *Clostridium thermoaceticum*), *Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui* und *Acetobacterium woodii* ausgewählt ist, wobei das flüssige Medium Milchsäure und/oder Salze davon in einer Konzentration in einem Bereich von 0,5 g/L bis 3 g/L umfasst, und wobei das gasförmige Substrat mindestens 10 Vol.-% CO₂ umfasst; und
Ermöglichen, dass der Mikroorganismus mindestens ein Produkt, ausgewählt aus Säuren und Alkoholen, herstellt.

2. Verwendung gemäß Anspruch 1, wobei das gasförmige Substrat ferner mindestens ein Gas, ausgewählt aus H₂, N₂ und CO, umfasst.

3. Verwendung gemäß Anspruch 1 oder 2, wobei das gasförmige Substrat mindestens 20 Vol.-% CO₂ umfasst.

4. Verwendung gemäß mindestens einem der vorhergehenden Ansprüche, wobei das mindestens eine Produkt aus Essigsäure, Propionsäure, Buttersäure, Acrylsäure und Fettsäure ausgewählt ist.

5. Verwendung gemäß mindestens einem der vorhergehenden Ansprüche, wobei das mindestens eine Produkt aus Ethanol, Aceton, Propanol, Butanol und 2,3-Butandiol ausgewählt ist.

6. Verwendung gemäß mindestens einem der vorhergehenden Ansprüche, wobei der Mikroorganismus zum Umwandeln von CO₂ und/oder CO in mindestens ein Produkt, ausgewählt aus Säuren und Alkoholen, in der Lage ist.

7. Verwendung von Milchsäure und/oder Salzen davon zum Induzieren von 1,5-fachem Anstieg oder mehr der Expression, relativ zu dem Mikroorganismus, welcher ohne die Gegenwart von Milchsäure und/oder Salzen davon kultiviert wird, von mindestens einem Gen, ausgewählt aus codH, codH beta, codH delta, codH gamma, coos1 und metF, in einem Verfahren von Verbessern der Effizienz von Kohlenstoffrückhaltung bei mikrobieller Fermentation, umfassend:
Bereitstellen eines gasförmigen Substrats und eines flüssigen Mediums für einen Mikroorganismus, welcher aus *Clostridium aceticum, Clostridium autoethanogenum, Clostridium carboxidivorans, Clostridium difficile, Clostridium ljungdahli, Moorella thermoacetica* (früher *Clostridium thermoaceticum*), *Methanobacterium thermoautotrophicum, Desulfobacterium autotrophicum, Clostridium sticklandii, Clostridium thermoautotrophicum, Clostridium formicoaceticum, Clostridium magnum, Acetobacterium carbinolicum, Acetobacterium kivui* und *Acetobacterium woodii* ausgewählt ist, wobei das flüssige Medium Milchsäure und/oder Salze davon in einer Konzentration in einem Bereich von 0,5 g/L bis 3 g/L umfasst, und wobei das gasförmige Substrat mindestens eine Kohlenstoffquelle, ausgewählt aus CO₂ und gegebenenfalls auch CO, umfasst, wobei das gasförmige Substrat mindestens 10 Vol.-% CO₂ umfasst; und
Ermöglichen, dass der Mikroorganismus mindestens ein Produkt, ausgewählt aus Säuren und Alkoholen, herstellt.

8. Verwendung gemäß Anspruch 7, wobei das gasförmige Substrat ferner mindestens ein Gas, ausgewählt aus H₂ und N₂, umfasst.

9. Verwendung gemäß Anspruch 7 oder 8, wobei das gasförmige Substrat mindestens 20 Vol.-% CO₂ umfasst.

10. Verwendung gemäß mindestens einem der vorhergehenden Ansprüche, wobei das mindestens eine Produkt aus Essigsäure, Propionsäure, Buttersäure, Acrylsäure und Fettsäure ausgewählt ist.

11. Verwendung gemäß mindestens einem der vorhergehenden Ansprüche, wobei das mindestens eine Produkt aus Ethanol, Aceton, Propanol, Butanol und 2,3-Butandiol ausgewählt ist.

12. Verwendung gemäß mindestens einem der vorhergehenden Ansprüche, wobei der Mikroorganismus zum Umwandeln von CO₂ und/oder CO in mindestens ein Produkt, ausgewählt aus Säuren und Alkoholen, in der Lage ist.

## Revendications

1. Utilisation de l'acide lactique et/ou de sels de celui-ci pour induire une augmentation de 1,5 ou plus de l'expression, par rapport au microorganisme cultivé sans la présence de l'acide lactique et/ou de sels de celui-ci, d'au moins un gène choisi parmi la codH (la monoxyde de carbone déshydrogénase/l'acétyl-coenzyme A synthase), la codH bêta (la sous-unité bêta de la monoxyde de carbone déshydrogénase/l'acétyl-coenzyme A synthase), la codH delta (la sous-unité delta de la monoxyde de carbone déshydrogénase/l'acétyl-coenzyme A synthase), la codH gamma (la sous-unité gamma de la monoxyde de carbone déshydrogénase/l'acétyl-coenzyme A synthase), la coos 1 (la monoxyde de carbone déshydrogénase de type anaérobie) et la metF (la méthylènetétrahydrofolate réductase) dans un procédé de fermentation microbienne d'un substrat gazeux comprenant du CO₂, le procédé comprenant les étapes consistant :
à fournir un substrat gazeux et un milieu liquide à un microorganisme choisi parmi le *Clostridium aceticum*, le *Clostridium autoethanogenum,* le *Clostridium carboxidivorans,* le *Clostridium difficile,* le *Clostridium Ijungdahli,* le *Moorella thermoacetica* (autrefois le *Clostridium thermoaceticum*), le *Methanobacterium thermoautotrophicum*, le *Desulfobacterium autotrophicum*, le *Clostridium sticklandii*, le *Clostridium thermoautotrophicum*, le *Clostridium formicoaceticum*, le *Clostridium magnum, l'Acetobacterium carbonilicum, l'Acetobacterium kivui* et *l'Acetobacterium woodii,* dans laquelle le milieu liquide comprend de l'acide lactique et/ou des sels de celui-ci à une concentration variant entre 0,5 g/l et 3 g/l et dans laquelle le substrat gazeux comprend au moins 10 % en volume de CO₂; et
à permettre au microorganisme de produire au moins un produit choisi parmi des acides et des alcools.

2. Utilisation selon la revendication 1, dans laquelle le substrat gazeux comprend en outre au moins un gaz choisi parmi le dihydrogène (H₂), le diazote (N₂) et le CO.

3. Utilisation selon la revendication 1 ou 2, dans laquelle le substrat gazeux comprend au moins 20 % en volume de CO₂.

4. Utilisation selon au moins une des revendications précédentes, dans laquelle le au moins un produit est choisi parmi l'acide acétique, l'acide propionique, l'acide butyrique, l'acide acrylique et l'acide gras.

5. Utilisation selon au moins une des revendications précédentes, dans laquelle le au moins un produit est choisi parmi l'éthanol, l'acétone, le propanol, le butanol et le 2,3-butanediol.

6. Utilisation selon au moins une des revendications précédentes, dans laquelle le microorganisme est capable de convertir le CO₂ et/ou le CO en au moins un produit choisi parmi des acides et des alcools.

7. Utilisation de l'acide lactique et/ou de sels de celui-ci pour induire une augmentation de 1,5 ou plus de l'expression, par rapport au microorganisme cultivé sans la présence de l'acide lactique et/ou de sels de celui-ci, d'au moins un gène choisi parmi la codH, la codH bêta, la codH delta, la codH gamma, la coos 1 et la metF dans un procédé d'amélioration de l'efficacité de capture du carbone lors d'une fermentation microbienne, comprenant les étapes consistant :
à fournir un substrat gazeux et un milieu liquide à un microorganisme choisi parmi le *Clostridium aceticum*, le *Clostridium autoethanogenum,* le *Clostridium carboxidivorans,* le *Clostridium difficile,* le *Clostridium Ijungdahli,* le *Moorella thermoacetica* (autrefois le *Clostridium thermoaceticum*), le *Methanobacterium thermoautotrophicum,* le *Desulfobacterium autotrophicum,* le *Clostridium sticklandii,* le *Clostridium thermoautotrophicum,* le *Clostridium formicoaceticum,* le *Clostridium magnum,* l'*Acetobacterium carbonilicum,* l'*Acetobacterium kivui* et l'*Acetobacterium woodii,* dans laquelle le milieu liquide comprend de l'acide lactique et/ou des sels de celui-ci à une concentration variant entre 0,5 g/l et 3 g/l et dans laquelle le substrat gazeux comprend au moins une source de carbone choisie parmi le CO₂, et facultativement également du CO, dans laquelle le substrat gazeux comprend au moins 10 % en volume de CO₂; et
à permettre au microorganisme de produire au moins un produit choisi parmi des acides et des alcools.

8. Utilisation selon la revendication 7, dans laquelle le substrat gazeux comprend en outre au moins un gaz choisi parmi le dihydrogène (H₂) et le diazote (N₂).

9. Utilisation selon la revendication 7 ou 8, dans laquelle le substrat gazeux comprend au moins 20 % en volume de CO₂.

10. Utilisation selon au moins une des revendications précédentes, dans laquelle le au moins un produit est choisi parmi l'acide acétique, l'acide propionique, l'acide butyrique, l'acide acrylique et l'acide gras.

11. Utilisation selon au moins une des revendications précédentes, dans laquelle le au moins un produit est choisi parmi l'éthanol, l'acétone, le propanol, le butanol et le 2,3-butanediol.

12. Utilisation selon au moins une des revendications précédentes, dans laquelle le microorganisme est capable de convertir le CO₂ et/ou le CO en au moins un produit choisi parmi des acides et des alcools.
